# FASCICULE DE BREVET EUROPEEN

(11) **EP 4 077 161 B1**
(45) Date de publication et mention de la délivrance du brevet: **12.07.2023**
(21) Numéro de dépôt: 20841969.7
(22) Date de dépôt: 15.12.2020
(51) Int. Cl.: B65D 83/08, G01N 33/487

(54) **DISPOSITIF DE STOCKAGE ET DE DISTRIBUTION DE BANDELETTES**
VORRICHTUNG ZUR LAGERUNG UND AUSGABE VON STREIFEN
DEVICE FOR STORING AND DISPENSING STRIPS

(30) Priorité: 16.12.2019 FR 1914454
(43) Date de publication de la demande: 26.10.2022
(73) Titulaire: BIOMÉRIEUX, 69280 Marcy L'Etoile (FR)
(72) Inventeur: JANUEL, Denis, 01500 AMBERIEU EN BUGEY (FR)
(74) Mandataire: bioMérieux PI Groupement mandataires
(86) Numéro de dépôt international: PCT/FR2020/000274
(87) Numéro de publication internationale: WO 2021/123516

(56) Documents cités:
- WO-A1-2014/134455
- DE-U1-202009 010 651
- US-A1- 2017 253 420

## Description

### Domaine technique de l'invention

L'invention concerne le domaine technique de l'analyse biologique. Plus particulièrement, l'invention porte sur les dispositifs contenant des bandelettes d'analyse.

Dans le domaine de l'analyse biologique, il peut être utilisé des bandelettes dites bandelettes d'analyse permettant de déterminer la concentration d'un ou plusieurs analytes dans les fluides biologiques ou détecter des faibles taux niveaux de résistance, confirmer ou détecter un phénotype de résistance spécifique ou encore confirmer un résultat d'antibiogramme. Ces bandelettes peuvent être des bandelettes « CMI » pour déterminer la Concentration Minimale Inhibitrice d'un antibiotique, d'un antifongique ou d'un antituberculeux, commercialisées notamment sous la marque ETEST^{®} par la société bioMérieux.

Pour préserver leur intégrité, ces bandelettes doivent être conservées à un certain taux d'humidité et doivent rester exemptes de substances étrangères. Ainsi, pour éviter toute contamination, ces bandelettes ne doivent pas être manipulées avant leur utilisation et doivent être maintenues dans des contenants hermétiques jusqu'à utilisation.

On connaît du document WO2012069758, l'usage de bandelettes d'analyse stériles à usage unique et emballées individuellement. Spécifiquement, dans ce document, il est décrit un emballage sécable dans lequel une bandelette d'analyse et un moyen de dessiccation sont insérés, le moyen de dessiccation constituant également un moyen de perforation de l'emballage pour accéder à la bandelette.

Ce type de dispositif présente l'inconvénient mineur de ne pouvoir contenir qu'une seule bandelette lorsqu'une analyse nécessite l'usage de plusieurs bandelettes.

Le document US2017/0253420 décrit un dispositif de stockage et de distribution de bandelettes d'analyse, dans lequel un organe de distribution permet d'extraire les bandelettes une à une par glissement.

### Objet de l'invention

L'invention a pour but d'améliorer le dispositif précédemment décrit et est notamment destiné à contenir, protéger et améliorer la préhension des bandelettes lorsque ces dernières sont rassemblées dans un même contenant.

A cet effet, l'invention a pour objet un dispositif de stockage et de distribution de bandelettes comprenant au moins une cassette comprenant une cavité avec un fond, au moins une première portion de la cavité étant conformée pour recevoir au moins une bandelette et préférentiellement une pluralité de bandelettes; ce dispositif comprend en outre au moins un organe de distribution configuré pour coopérer avec la cassette, ledit organe de distribution étant au moins partiellement logé dans une deuxième portion de la cavité de la cassette, la première portion de la cavité et la deuxième portion de la cavité étant reliées par une portion intermédiaire inclinée, la deuxième portion de la cavité étant plus profonde que la première portion par rapport à un axe sensiblement perpendiculaire au fond de la cavité; l'organe de distribution comprend une partie saillante présentant une surface de contact conformée pour recouvrir au moins une première extrémité de chaque bandelette, la surface de contact étant en regard du fond de la cavité de la cassette et surplombant la portion intermédiaire inclinée, ledit organe de distribution étant configuré pour au moins être mobile en rotation entre :
- une position de repos, dans laquelle la partie saillante s'étend à distance de la portion intermédiaire inclinée,
- une position de fonctionnement, dans laquelle la partie saillante converge vers la portion intermédiaire de sorte à au moins plaquer la première extrémité de chaque bandelette contre la portion intermédiaire inclinée.

Le dispositif selon l'invention permet de contenir une pluralité de bandelettes et de faciliter la préhension des bandelettes. En effet, en position de fonctionnement, l'organe de distribution converge vers la portion intermédiaire inclinée de manière à ce que au moins la première extrémité de chaque bandelette recouverte par la surface de contact de la partie saillante de l'organe de distribution soit plaquée contre la portion intermédiaire inclinée et préférentiellement soit pincée entre la partie saillante et la portion intermédiaire inclinée. Le plaquage/pincement de la première extrémité de chaque bandelette entraine le basculement de ces dernières, les disposant en éventail pour permettre la préhension individuelle de chacune.

Selon une caractéristique additionnelle de l'invention, l'organe de distribution est un organe de dessiccation, ce qui permet d'assurer la protection de toutes les bandelettes contre l'humidité pendant toute sa durée de vie, et ce, quel que soit le nombre de bandelettes au sein du dispositif de stockage. Ainsi, selon une caractéristique de l'invention, l'organe de distribution est composé de matière plastique et d'un matériau dessiccatif.

Selon une caractéristique additionnelle de l'invention, l'organe de distribution passe de la position de repos à la position de fonctionnement par l'exercice d'une pression sur la partie saillante de l'organe de distribution. Préférentiellement, la pression est exercée au niveau de la partie saillante de l'organe de distribution. Cette pression permet de relever les bandelettes et de les récupérer avec un instrument de préhension manuelle.

Selon une caractéristique additionnelle de l'invention, l'organe de distribution est logé à demeure dans la cassette.

Selon une caractéristique additionnelle de l'invention, la cassette est de forme sensiblement rectangulaire.

Selon une caractéristique additionnelle de l'invention, la cavité de la cassette est longitudinale, préférentiellement centrée dans la cassette. Avantageusement, la cavité de la cassette est conformée pour loger les bandelettes de manière ajustée. Ainsi, les bandelettes restent en positon et sont empêchées de se déplacer horizontalement.

Selon une caractéristique additionnelle de l'invention, la partie du fond de la cavité au niveau de la deuxième portion est décalée vers le bas selon l'axe D-D sensiblement perpendiculaire au fond.

Avantageusement, la portion intermédiaire inclinée présente une pente comprise entre 25° et 35°.

La cassette peut comprendre des retraits latéraux ménagés de part et d'autre de la cassette et configurés pour permettre le maintien en position dans l'outil de prise des bandelettes.

En outre, selon une caractéristique additionnelle de l'invention, la cassette comprend au moins une patte de retenue positionnée à une extrémité de la cavité de la cassette. Préférentiellement, la patte de retenue est ménagée sur la première portion de la cavité.

La patte de retenue est configurée pour empêcher la sortie inopinée de plusieurs bandelettes en même temps hors de la cavité, notamment lors de l'utilisation du dispositif de stockage dans une machine automatique.

Selon une caractéristique additionnelle de l'invention, le dispositif de stockage comprend un opercule de scellage appliqué, préférentiellement soudé, sur la cassette. L'opercule permet de rendre étanche la cassette, avant la première utilisation. Avantageusement, l'opercule de scellage est préférentiellement en aluminium.

Selon une caractéristique additionnelle de l'invention, le dispositif comprend un couvercle destiné à coopérer avec la cassette. Avantageusement, le couvercle sert de relai de protection étanche, une fois l'opercule de scellage ouvert.

Selon une caractéristique additionnelle de l'invention, le couvercle présente un rebord circonférentiel destiné à coopérer avec la cassette par complémentarité de forme et préférentiellement par clippage.

Selon une caractéristique additionnelle de l'invention, le couvercle comprend une face interne destinée à être en regard de la cassette, ladite face interne comprenant au moins une nervure de retenue s'étendant longitudinalement sur ladite face interne et destinée à être agencée au moins partiellement dans la cavité, et préférentiellement de la première portion de la cavité de la cassette, lorsque le couvercle coopère avec la cassette. Avantageusement, la ou les nervures de retenue permettent de maintenir les bandelettes dans la cavité de la cassette et donc d'éviter le déplacement ou le retournement de ces dernières pendant le transport du dispositif de stockage.

Selon une caractéristique additionnelle de l'invention, le couvercle comprend au moins un joint surmoulé ou bi-injecté sur la face interne du couvercle pour améliorer l'étanchéité.

Selon une caractéristique additionnelle de l'invention, le joint est conformé pour coopérer avec la forme de la cavité. Avantageusement, le joint se présente sous la forme d'un anneau allongé ou d'un cordon sous forme d'anneau.

Selon une caractéristique additionnelle de l'invention, le couvercle comprend un détrompeur, afin d'indiquer le sens de fermeture. Avantageusement, le détrompeur est agencé au niveau d'un organe de fermeture de la cassette.

Avantageusement, la forme de l'organe de distribution est optimisée afin de permettre le fonctionnement mécanique de ce dernier tout en conservant un maximum de capacité d'adsorption d'humidité.

Selon une caractéristique additionnelle de l'invention, l'organe de distribution comprend une deuxième partie qui est élargie par rapport à la partie saillante afin de garantir sa position dans la cassette et augmenter son volume et donc son pouvoir dessicant.

Selon une caractéristique additionnelle de l'invention, la partie saillante s'étend radialement par rapport à la deuxième partie.

Selon une caractéristique additionnelle de l'invention, l'organe de distribution est évidé de manière à optimiser le moulage dudit organe et également à optimiser la surface d'absorption.

Selon une caractéristique additionnelle de l'invention, l'organe de distribution présente une section transversale avant-arrière le long de l'axe longitudinal de forme sensiblement triangulaire. Cette forme permet de favoriser le basculement de l'organe de distribution vers l'avant, c'est-à-dire vers la partie saillante.

Selon une caractéristique additionnelle de l'invention, chaque bandelette comprend une première extrémité destinée à être retenue par la partie saillante de l'organe de distribution.

Par ailleurs, selon une caractéristique additionnelle de l'invention, chaque bandelette comprend une deuxième extrémité, opposée à la première extrémité, destinée à être retenue par une patte de retenue.

Avantageusement, la ou les bandelettes sont des bandelettes d'analyse permettant de déterminer la concentration d'un ou plusieurs analytes dans les fluides biologiques ou détecter des faibles taux niveaux de résistance, confirmer ou détecter un phénotype de résistance spécifique ou encore confirmer un résultat d'antibiogramme. Ces bandelettes peuvent être des bandelettes « CMI » pour déterminer la Concentration Minimale Inhibitrice d'un antibiotique, d'un antifongique ou d'un antituberculeux, commercialisées notamment sous la marque ETEST^{®} par la société bioMérieux.

Selon une caractéristique additionnelle de l'invention, le couvercle comprend en outre au moins une nervure transversale de maintien, agencée sur la face interne du couvercle en regard de la cavité et configurée pour permettre le maintien des bandelettes au sein de la cavité lorsque le couvercle est positionné sur la cassette.

L'invention a également pour objet un ensemble, comprenant au moins une bandelette d'analyse, préférentiellement une pluralité de bandelettes d'analyse et un dispositif de stockage et de distribution selon l'une quelconque des revendications précédentes, l'au moins une bandelette d'analyse étant logée dans la première partie de la cavité du dispositif de stockage, positionnée à plat par rapport au fond de la cavité, selon l'axe longitudinal du dispositif.

Selon une caractéristique additionnelle de l'invention, en position de repos de l'organe de distribution, la première extrémité de chaque bandelette repose sous la surface de contact de la partie saillante de l'organe de distribution.

Selon une caractéristique additionnelle de l'invention, en position de fonctionnement de l'organe de distribution, la première extrémité de chaque bandelette est pincée entre la partie saillante de l'organe de distribution et la portion intermédiaire inclinée.

Dans la présente invention, on entend par organe de distribution, un organe réalisé au moins en partie dans un matériau contenant un tamis moléculaire ou un agent chimique permettant l'absorption d'eau/d'humidité.

### Brève description des figures

L'invention sera mieux comprise, grâce à la description ci-après, qui se rapporte à un mode de réalisation selon la présente invention, donné à titre d'exemple non limitatif et expliqué avec référence aux figures schématiques annexées. Les figures schématiques annexées sont listées ci-dessous :
La figure 1 est une vue en perspective de l'ensemble fermé hermétiquement selon l'invention,
La figure 2 est une vue en perspective de l'ensemble fermé hermétiquement sans couvercle,
La figure 3 est une vue en perspective de l'ensemble ouvert,
La figure 4 est une vue en perspective du couvercle vue de dessous,
La figure 5 est une vue de dessus de la cassette selon l'invention,
La figure 6 est une vue en coupe longitudinale de la cassette représentée en figure 5, selon l'axe A-A,
La figure 7 est une vue en perspective de dessus de l'organe de distribution,
La figure 8 est une vue en perspective de dessous de l'organe de distribution,
La figure 9 est une vue en coupe transversale avant-arrière selon un axe B-B de l'organe de distribution représenté en figures 7 et 8,
La figure 10 est une vue en coupe longitudinale du dispositif selon l'invention, l'organe de distribution étant en position de repos,
La figure 11 est une vue en coupe longitudinale du dispositif selon l'invention, l'organe de distribution étant en position de fonctionnement,
La figure 12 est une vue en perspective du dispositif selon l'invention, l'organe de distribution étant en position de fonctionnement,
La figure 13 est une vue en perspective du dispositif selon l'invention, un instrument de préhension étant mis en place pour la préhension d'une bandelette.

### Description détaillée

Dans l'exemple illustré aux figures, le dispositif 1 de stockage selon l'invention comprend une cassette 10, recouverte d'un couvercle 11, un organe de distribution 12. Ce dispositif de stockage fait partie d'un ensemble comprenant en outre une pluralité de bandelettes 13 destinées à être logées dans la cassette 10 du dispositif 1.

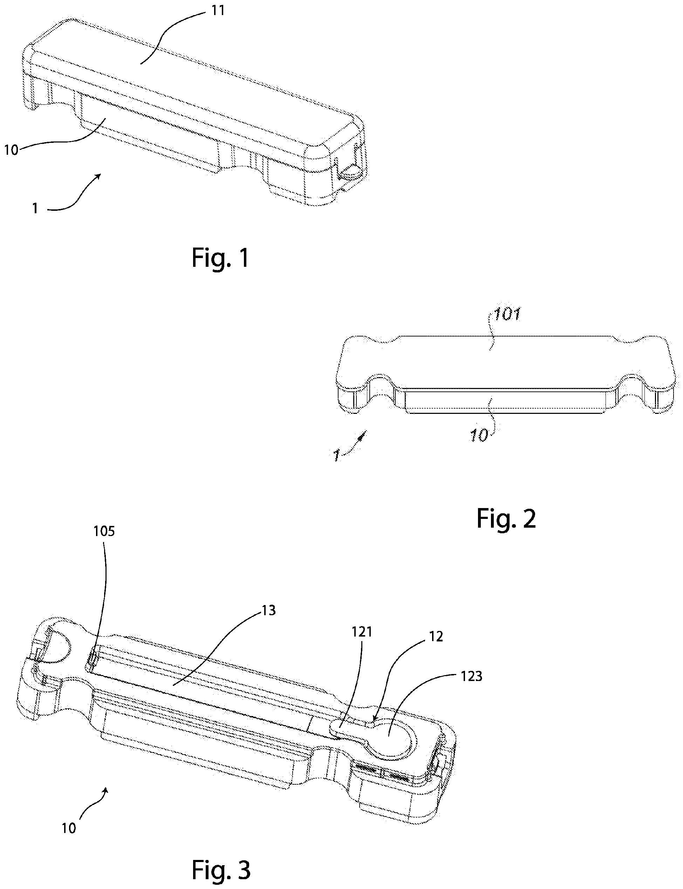

En figure 1 est illustré le dispositif de stockage 1 fermé. En figure 2, l'ensemble 1 est ouvert et le couvercle 11 a été retiré. Sur cette figure, on peut donc voir la cassette 10 recouverte d'un opercule 101 permettant de maintenir l'étanchéité de la cassette bien que le couvercle 11 ait été retiré. L'opercule 101 recouvre la totalité de la face supérieure de la cassette 10.

En ouvrant l'opercule 101 comme illustré en figure 3, on découvre la cassette 10, comprenant une cavité 102 dans laquelle est logé une pluralité de bandelettes 13 d'analyse, et un organe de distribution 12.

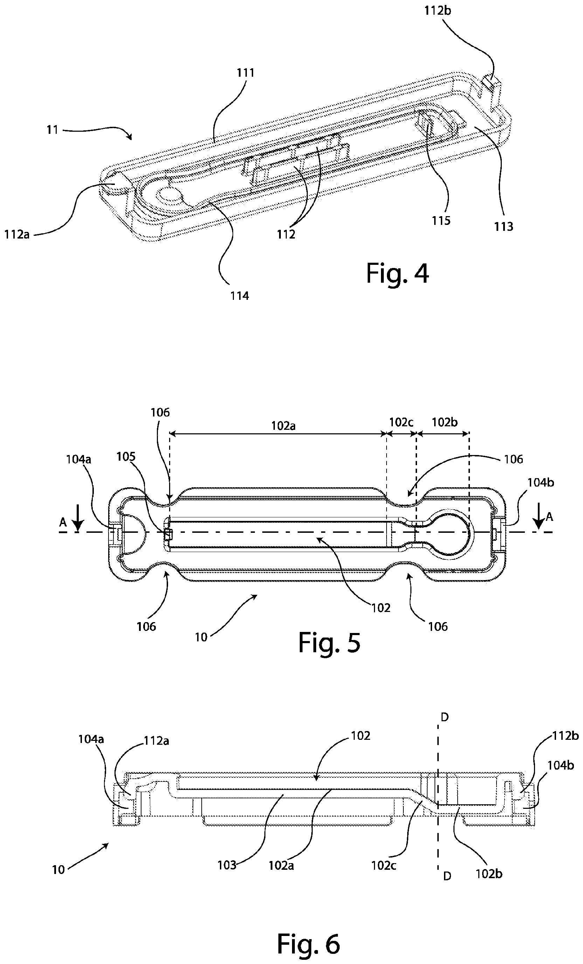

Le couvercle 11 de l'ensemble 1 est illustré plus en détail en figure 4. Le couvercle présente un rebord circonférentiel 111 destiné à coopérer avec la cassette 10, par complémentarité de forme et préférentiellement par clippage. Plus particulièrement, le couvercle comprend une première languette 112a à une extrémité destinée à coopérer avec un premier orifice d'engagement 104a ménagé sur la cassette 10, et une deuxième languette 112b positionnée à l'extrémité opposée et destinée à coopérer avec un deuxième orifice d'engagement 104b ménagé sur la cassette 10.

Avantageusement, on appelle détrompeur le fait que le premier orifice d'engagement 104a et le deuxième orifice d'engagement 104b ne soient pas identiques. En effet, chacun présente une ouverture de dimension différente de sorte que le couvercle 11, ne puisse s'engager via respectivement la première languette 112a et la deuxième languette 112b que dans un sens sur la cassette 10. A ces fins, la première languette 112a du couvercle 11 ne peut coopérer qu'avec le premier orifice d'engagement 104a et la deuxième languette 112b du couvercle 11 ne peut s'engager qu'avec le deuxième orifice d'engagement 104b.

En outre, le couvercle 11 comprend une face 113 destinée à être en regard de la cassette 10, ladite face interne 113 comprenant deux nervures 112 s'étendant longitudinalement sur ladite face interne 113. Les deux nervures 112 sont disposées parallèlement l'une à l'autre et préférentiellement sensiblement au milieu de la face interne 113 du couvercle 11. Les deux nervures 112 sont positionnées de manière à pouvoir maintenir les bandelettes 13 dans la cavité 102 de la cassette 10 lorsque le couvercle 11 recouvre la cassette 10. Dans une variante non représentée, la face interne 113 présente une seule nervure 112 disposée transversalement et sensiblement au milieu de ladite face interne 113.

Par ailleurs, le couvercle 11 comprend un joint 114 présentant une forme sensiblement complémentaire à une cavité longitudinale 102 ménagée dans la cassette 10 et configurée pour loger les bandelettes 13. Avantageusement, le joint 114 est rapporté sur la face interne 113 du couvercle 11, comme visible en figure 4.

En outre, comme on peut le voir notamment en figure 4, le couvercle 11 comprend en outre au moins une nervure transversale de maintien 115, agencée sur la face interne du couvercle 11 en regard de la cavité longitudinale 12 et configurée pour permettre le maintien des bandelettes au sein de la cavité lorsque le couvercle 11 est positionné sur la cassette 10.

La cassette 10 selon l'invention est de forme sensiblement rectangulaire. La cassette 10 comprend une cavité 102 longitudinale, préférentiellement centrée, comme on peut le voir notamment en figure 5. La cavité 102 comprend un fond 103. En outre, la cavité 102 comprend une première portion 102a conformée pour recevoir au moins une bandelette 13 et préférentiellement une pluralité de bandelettes 13, et une deuxième portion 102b conformée pour loger au moins en partie l'organe de distribution 12 comme illustré en figure 6.

Dans l'exemple illustré en figure 6, la première portion 102a de la cavité et la deuxième portion 102b de la cavité 102 sont reliées par une portion intermédiaire inclinée 102c, la deuxième portion 102b de la cavité 102 étant plus profonde que la première portion 102a par rapport à un axe D-D sensiblement perpendiculaire au fond 103 de la cavité 102. Autrement dit, la partie du fond 103 de la cavité 102 au niveau de la deuxième portion 102b est décalée selon l'axe D-D sensiblement perpendiculaire au fond 103, vers le bas c'est-à-dire à l'opposé de la face supérieure de la cassette 10.

Avantageusement, la portion intermédiaire inclinée présente une pente comprise entre 25 ° et 35 °.

Par ailleurs, comme on peut le voir notamment en figure 5, la cassette 10 comprend un premier orifice d'engagement 104a ménagé sur une première extrémité de la cassette et destiné à coopérer avec la première languette 112a du couvercle 11. La cassette 10 comprend également un deuxième orifice d'engagement 104b sur une deuxième extrémité, opposée à la première extrémité, de la cassette et destiné à coopérer avec la deuxième languette 112b du couvercle 11.

Selon l'invention, la cassette 10 peut comprendre des retraits latéraux 106 ménagés de manière symétrique de part et d'autre de la cassette et configurés pour permettre le maintien en position dans l'outil de prise des bandelettes.

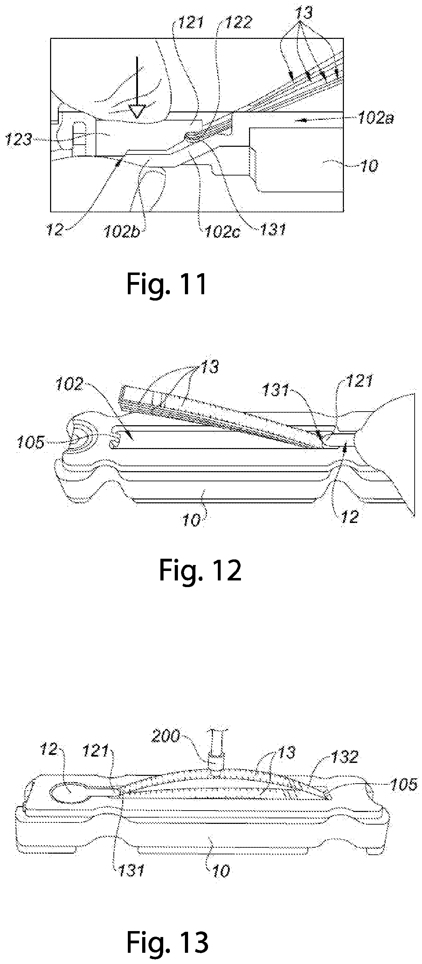

En outre, la cassette 10 comprend au moins une patte de retenue 105 positionnée à une extrémité de la cavité 102 de la cassette 10. La patte de retenue 105 étant ménagée sur la première portion 102a de la cavité 102. La patte de retenue 105 est configurée pour empêcher les bandelettes de sortir de la cavité 102. La patte de retenue 105 permet également de faciliter la préhension au milieu de la bandelette par un instrument, comme visible en figure 13. La patte de retenue 105 maintient la deuxième extrémité 132 des bandelettes 13.

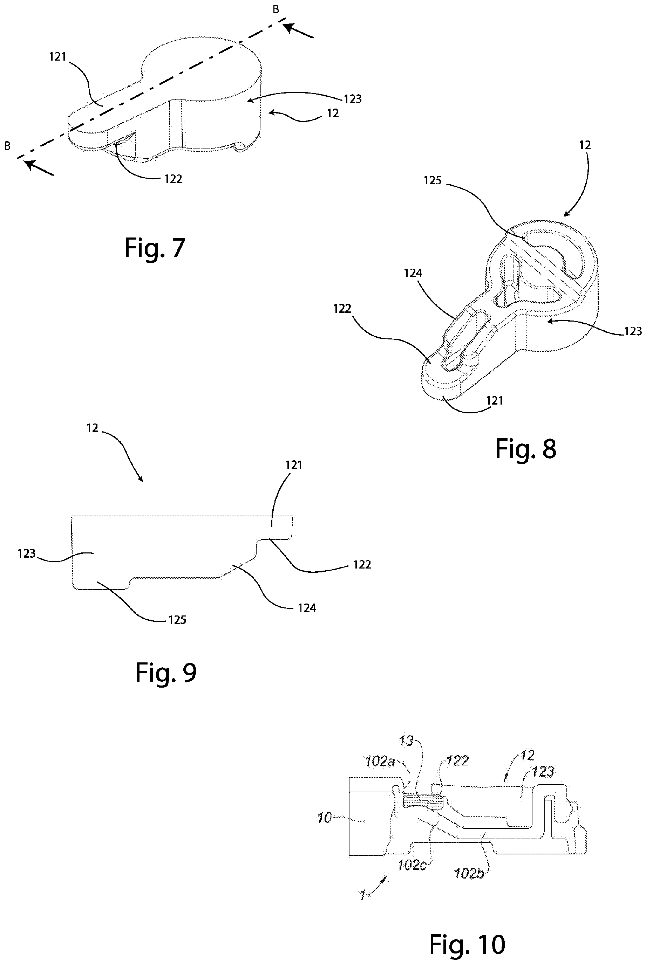

L'organe de distribution 12 est illustré notamment aux figures 7 à 9. L'organe de distribution est configuré pour coopérer avec la cassette 10, ledit organe de distribution 12 étant au moins partiellement logé dans une deuxième portion 102b de la cavité 102 de la cassette 10, comme illustré aux figures 10 et 11 notamment.

Comme on peut le voir aux figures 7 et 8, l'organe de distribution 12 comprend une partie saillante 121 présentant une surface de contact 122 conformée pour recouvrir au moins une première extrémité 131 de chaque bandelette 13, la surface de contact 122 étant en regard du fond 103 de la cavité 102 de la cassette 10 et surplombant la portion intermédiaire inclinée 102c.

En outre, l'organe de distribution 12 comprend une deuxième partie 123 qui est élargie par rapport à la partie saillante 121. Plus particulièrement, la partie saillante 121 s'étend radialement ou latéralement (suivant la forme de la deuxième partie) par rapport à la deuxième partie 123.

En outre, l'organe de distribution présente une section transversale avant-arrière le long de l'axe longitudinal de forme sensiblement triangulaire, comme illustré en figure 9. Cette forme permet de favoriser le basculement de l'organe de distribution 12 vers l'avant, c'est-à-dire vers la partie saillante 121. En effet, comme on peut le voir en figures 8 et 9, la deuxième partie 123 de l'organe de distribution 12 présente une surface d'appui 125 suivie d'un épaulement suivi d'une surface plane sensiblement parallèle à la surface d'appui 125. Une portion inclinée 124 relie la surface plane à la partie saillante 121 de l'organe de distribution 12.

Le but de l'invention est notamment de faciliter la préhension individuelle des bandelettes rassemblées dans un même contenant. Pour ce faire, l'organe de distribution 12 est configuré pour être mobile en rotation entre :
une position de repos illustrée en figure 10, dans laquelle la partie saillante 121 s'étend à distance de la portion intermédiaire inclinée 102c, et
une position de fonctionnement illustrée en figures 11, 12, 13, dans laquelle la partie saillante 121 converge vers la portion intermédiaire inclinée 102c, de manière à ce que la première extrémité 131 de chaque bandelette 13 soit au moins plaquée contre la portion intermédiaire inclinée 102c, et préférentiellement pincée entre la partie saillante 121 et la portion intermédiaire inclinée 102c.

Comme on peut le voir en figure 10, les bandelettes 13 sont recouvertes sur une première extrémité 131 par la partie saillante 121 de l'organe de distribution 12. Lors du basculement/de la rotation de l'organe de distribution 12, en position de fonctionnement, la partie saillante 121 appuie sur l'extrémité 131 des bandelettes contre la portion intermédiaire inclinée 102c de la cassette, engendrant alors un basculement des bandelettes comme illustré en figure 12. Lorsque la pression exercée sur l'organe de distribution est réalisée manuellement, la patte de retenue 105 n'est plus en mesure de retenir la deuxième extrémité 132 des bandelettes 13, ce qui permet ainsi une préhension manuelle et individuelle desdites bandelettes 13.

Par ailleurs, lorsque la pression exercée sur l'organe de distribution est moins importante qu'une pression manuelle standard, la patte de retenue 105 retient la deuxième extrémité 132 des bandelettes 13, engendrant la courbure des bandelettes 13, comme illustré en figure 13, ce qui permet une préhension par un instrument 200 de type automatique par exemple par aspiration du centre de la bandelette 13. De plus, cette courbure permet de créer un mouvement de friction entre la bandelette saisie et la bandelette située directement dessous et qui pourrait être collée et de les séparer.

Dans les deux cas, le basculement total ou la courbure des bandelettes permet le décollement des bandelettes entre elles et permet la saisie d'une seule bandelette à la fois, et ce quel que soit l'instrument utilisé. Cela permet en outre de s'assurer que la bandelette située dessous reste en place dans la cassette et ne se retourne pas.

En figure 12, un instrument manuel ou la main d'un opérateur peut être utilisé, tandis qu'en figure 13, un instrument automatique 200 (ventouse ou tout autre système de préhension) peut être utilisé.

Bien entendu, l'invention n'est pas limitée aux modes de réalisation décrits et représentés aux figures annexées. Des modifications restent possibles, notamment du point de vue de la constitution des divers éléments ou par substitution d'équivalents techniques, sans sortir pour autant du domaine de protection de l'invention.

## Revendications

1. Dispositif de stockage (1) et de distribution de bandelettes (13) comprenant au moins une cassette (10) comprenant une cavité (12) avec un fond (103), au moins une première portion de la cavité (102) étant conformée pour recevoir au moins une bandelette (13) et préférentiellement une pluralité de bandelettes (13), le dispositif de stockage (1) et de distribution comprenant en outre au moins un organe de distribution (12) configuré pour coopérer avec la cassette (10), ledit organe de distribution (12) étant au moins partiellement logé dans une deuxième portion de la cavité de la cassette (10), l'organe de distribution (12) comprenant une partie saillante (121) présentant une surface de contact (122) conformée pour recouvrir au moins une première extrémité (131) de chaque bandelette (13), la surface de contact (122) étant en regard du fond (103) de la cavité (102) de la cassette (10) **caractérisé en ce que** la première portion de la cavité (102) et la deuxième portion de la cavité (102) sont reliées par une portion intermédiaire inclinée (102c), la deuxième portion de la cavité (102) étant plus profonde que la première portion de la cavité (102) par rapport à un axe sensiblement perpendiculaire D-D au fond (103) de la cavité (102) et **en ce que** la surface de contact (122) de l'organe de distribution (12) surplombe la portion intermédiaire inclinée (102c), ledit organe de distribution (12) étant configuré pour au moins être mobile en rotation entre :
- une position de repos, dans laquelle la partie saillante (121) s'étend à distance de la portion intermédiaire inclinée (102c),
- une position de fonctionnement, dans laquelle la partie saillante (121) converge vers la portion intermédiaire inclinée (102c) de sorte à au moins plaquer la première extrémité (131) de chaque bandelette (13) contre la portion intermédiaire inclinée (102c).

2. Dispositif de stockage et de distribution selon la revendication 1, dans lequel l'organe de distribution (12) est un organe de dessiccation.

3. Dispositif de stockage et de distribution selon l'une quelconque des revendications 1 ou 2, dans lequel la portion intermédiaire inclinée (102c) présente une pente comprise entre 25 ° et 35 °.

4. Dispositif de stockage et de distribution selon l'une quelconque des revendications 1 à 3, dans lequel la cassette (10) comprend au moins une patte de retenue (105) positionnée à une extrémité de la cavité (102) de la cassette (10).

5. Dispositif de stockage et de distribution selon l'une quelconque des revendications 1 à 4, dans lequel l'organe de distribution (12) comprend une deuxième partie (123) qui est élargie par rapport à la partie saillante (121), la partie saillante (121) s'étendant radialement par rapport à la deuxième partie (123).

6. Dispositif de stockage et de distribution selon l'une quelconque des revendications 1 à 5, dans lequel l'organe de distribution (12) présente une section transversale avant-arrière le long de l'axe longitudinal B-B de forme sensiblement triangulaire.

7. Dispositif de stockage et de distribution selon l'une quelconque des revendications 1 à 6, comprenant un couvercle (11) destiné à coopérer avec la cassette (10).

8. Dispositif de stockage et de distribution selon la revendication 7, dans lequel le couvercle (11) comprend une face interne (113) destinée à être en regard de la cassette (10), ladite face interne (113) comprenant au moins une nervure de retenue (112) s'étendant longitudinalement sur ladite face interne (113) et destinée à être agencée au moins partiellement dans la cavité (102), et préférentiellement de la première portion de la cavité (102) de la cassette (10), lorsque le couvercle (11) coopère avec la cassette (10).

9. Ensemble, comprenant au moins une bandelette (13) d'analyse, préférentiellement une pluralité de bandelettes (13) d'analyse et un dispositif de stockage et de distribution (1) selon l'une quelconque des revendications précédentes, l'au moins une bandelette (13) d'analyse étant logée dans la première partie de la cavité (102) du dispositif (1), positionnée à plat par rapport au fond (103) de la cavité (102), selon l'axe longitudinal du dispositif (1).

10. Ensemble selon la revendication 9, dans lequel en position de repos de l'organe de distribution (12), la première extrémité (131) de chaque bandelette (13) repose sous la surface de contact (122) de la partie saillante (121) de l'organe de distribution (12).

11. Ensemble selon l'une quelconque des revendications 9 ou 10, dans lequel en position de fonctionnement de l'organe de distribution (12), la première extrémité (131) de chaque bandelette (13) est au moins plaquée contre la portion intermédiaire inclinée (102c) et préférentiellement pincée entre la partie saillante (121) de l'organe de distribution (12) et la portion intermédiaire inclinée (102c).

## Patentansprüche

1. Vorrichtung (1) zur Lagerung und Ausgabe von Steifen (13) mit mindestens einer Kassette (10), die einen Hohlraum (102) mit einem Boden (103) umfasst, wobei zumindest ein erster Abschnitt des Hohlraums (102) so geformt ist, dass er mindestens einen Steifen (13) und vorzugsweise mehrere Steifen (13), aufnehmen kann,
wobei die Lager- und Ausgabevorrichtung (1) ferner mindestens ein Ausgabeorgan (12) umfasst, das dazu konfiguriert ist, mit der Kassette (10) zusammenzuwirken, wobei das Ausgabeorgan (12) zumindest teilweise in einem zweiten Abschnitt des Hohlraums der Kassette (10) untergebracht ist,
wobei das Ausgabeorgan (12) einen vorstehenden Teil (121) umfasst, der eine Kontaktfläche (122) aufweist, die so geformt ist, dass sie zumindest ein erstes Ende (131) jedes Steifens (13) bedeckt, wobei die Kontaktfläche (122) dem Boden (103) des Hohlraums (102) der Kassette (10) zugewandt ist,
**dadurch gekennzeichnet, dass** der erste Abschnitt des Hohlraums (102) und der zweite Abschnitt des Hohlraums (102) durch einen geneigten Zwischenabschnitt (102c) verbunden sind, wobei der zweite Abschnitt des Hohlraums (102) in Bezug auf eine zum Boden (103) des Hohlraums (102) im Wesentlichen senkrechte Achse D-D tiefer als der erste Abschnitt des Hohlraums (102) ist, und dass die Kontaktfläche (122) des Ausgabeorgans (12) den geneigten Zwischenabschnitt (102c) überragt, wobei das Ausgabeorgan (12) dazu konfiguriert ist, zumindest drehbeweglich zu sein zwischen:
- einer Ruheposition, in der sich der vorstehende Teil (121) vom geneigten Zwischenabschnitt (102c) weg erstreckt,
- einer Betriebsposition, in der der vorstehende Teil (121) in Richtung des geneigten Zwischenabschnitts (102c) konvergiert, sodass das erste Ende (131) jedes Steifens (13) zumindest gegen den geneigten Zwischenabschnitt (102c) gedrückt wird.

2. Lager- und Ausgabevorrichtung nach Anspruch 1, wobei das Ausgabeorgan (12) ein Trocknungsorgan ist.

3. Lager- und Ausgabevorrichtung nach einem der Ansprüche 1 oder 2, wobei der geneigte Zwischenabschnitt (102c) eine Neigung zwischen 25° und 35° aufweist.

4. Lager- und Ausgabevorrichtung nach einem der Ansprüche 1 bis 3, wobei die Kassette (10) mindestens eine Haltelasche (105) umfasst, die an einem Ende des Hohlraums (102) der Kassette (10) positioniert ist.

5. Lager- und Ausgabevorrichtung nach einem der Ansprüche 1 bis 4, wobei das Ausgabeorgan (12) einen zweiten Teil (123) umfasst, der in Bezug auf den vorstehenden Teil (121) verbreitert ist, wobei sich der vorstehende Teil (121) in Bezug auf den zweiten Teil (123) radial erstreckt.

6. Lager- und Ausgabevorrichtung nach einem der Ansprüche 1 bis 5, wobei das Ausgabeorgan (12) einen Querschnitt vor vorn nach hinten entlang der Längsachse B-B mit einer im Wesentlichen dreieckigen Form aufweist.

7. Lager- und Ausgabevorrichtung nach einem der Ansprüche 1 bis 6, umfassend einen Deckel (11), der dazu bestimmt ist, mit der Kassette (10) zusammenzuwirken.

8. Lager- und Ausgabevorrichtung nach Anspruch 7, wobei der Deckel (11) eine Innenseite (113) umfasst, die dazu bestimmt ist, der Kassette (10) zugewandt zu sein, wobei die Innenseite (113) mindestens eine Rückhalterippe (112) umfasst, die sich in Längsrichtung an der Innenseite (113) erstreckt und dazu bestimmt ist, zumindest teilweise im Hohlraum (102) angeordnet zu sein, und vorzugsweise dem ersten Abschnitt des Hohlraums (102) der Kassette (10), wenn der Deckel (11) mit der Kassette (10) zusammenwirkt.

9. Baugruppe mit mindestens einem Teststeifen (13), vorzugsweise mehreren Teststeifen (13), und einer Lager- und Ausgabevorrichtung (1) nach einem der vorangehenden Ansprüche, wobei der mindestens eine Teststeifen (13) im ersten Teil des Hohlraums (102) der Vorrichtung (1) angeordnet ist, in Bezug auf den Boden (103) des Hohlraums (102) entlang der Längsachse der Vorrichtung (1) flach positioniert.

10. Baugruppe nach Anspruch 9, wobei in der Ruheposition des Ausgabeorgans (12) das erste Ende (131) jedes Steifens (13) auf der Kontaktfläche (122) des vorstehenden Teils (121) des Ausgabeorgans (12) aufliegt.

11. Baugruppe nach einem der Ansprüche 9 oder 10, wobei in der Betriebsposition des Ausgabeorgans (12) das erste Ende (131) jedes Steifens (13) zumindest gegen den geneigten Zwischenabschnitt (102c) gedrückt wird und vorzugsweise zwischen dem vorstehenden Teil (121) des Ausgabeorgans (12) und dem geneigten Zwischenabschnitt (102c) eingeklemmt ist.

## Claims

1. Device (1) for storing and dispensing strips (13), comprising at least one cartridge (10) comprising a cavity (102) with a bottom (103), at least a first portion of the cavity (102) being shaped to receive at least one strip (13) and preferably a plurality of strips (13),
the storage and dispensing device (1) further comprising at least one dispensing member (12) configured to cooperate with the cartridge (10), said dispensing member (12) being at least partially accommodated in a second portion of the cavity of the cartridge (10),
the dispensing member (12) comprising a protruding part (121) having a contact surface (122) shaped to cover at least a first end (131) of each strip (13), the contact surface (122) facing towards the bottom (103) of the cavity (102) of the cartridge (10), **characterized in that** the first portion of the cavity (102) and the second portion of the cavity (102) are connected by an inclined intermediate portion (102c), the second portion of the cavity (102) being deeper than the first portion of the cavity (102) with respect to an axis D-D substantially perpendicular to the bottom (103) of the cavity (102), and **in that** the contact surface (122) of the dispensing member (12) overhangs the inclined intermediate portion (102c), said dispensing member (12) being configured to at least be mobile in rotation between:
- a resting position, in which the protruding part (121) extends at a distance from the inclined intermediate portion (102c),
- an operating position, in which the protruding part (121) converges towards the inclined intermediate portion (102c) so as to at least press the first end (131) of each strip (13) against the inclined intermediate portion (102c).

2. Storage and dispensing device according to Claim 1, wherein the dispensing member (12) is a desiccating member.

3. Storage and dispensing device according to either one of Claims 1 and 2, wherein the inclined intermediate portion (102c) has a slope of between 25° and 35°.

4. Storage and dispensing device according to any one of Claims 1 to 3, wherein the cartridge (10) comprises at least one retaining tab (105) positioned at one end of the cavity (102) of the cartridge (10).

5. Storage and dispensing device according to any one of Claims 1 to 4, wherein the dispensing member (12) comprises a second part (123), which is widened with respect to the protruding part (121), the protruding part (121) extending radially with respect to the second part (123) .

6. Storage and dispensing device according to any one of Claims 1 to 5, wherein the dispensing member (12) has a front-to-rear cross section of substantially triangular shape along the longitudinal axis B-B.

7. Storage and dispensing device according to any one of Claims 1 to 6, comprising a cover (11) intended to cooperate with the cartridge (10).

8. Storage and dispensing device according to Claim 7, wherein the cover (11) comprises an internal face (113) intended to face towards the cartridge (10), said internal face (113) comprising at least one retaining rib (112) extending longitudinally over said internal face (113) and intended to be arranged at least partially in the cavity (102), and preferably the first portion of the cavity (102) of the cartridge (10), when the cover (11) cooperates with the cartridge (10).

9. Assembly comprising at least one analysis strip (13), preferably a plurality of analysis strips (13), and a storage and dispensing device (1) according to any one of the preceding claims, the at least one analysis strip (13) being accommodated in the first part of the cavity (102) of the device (1), positioned flat with respect to the bottom (103) of the cavity (102), along the longitudinal axis of the device (1).

10. Assembly according to Claim 9, wherein in the resting position of the dispensing member (12), the first end (131) of each strip (13) rests under the contact surface (122) of the protruding part (121) of the dispensing member (12).

11. Assembly according to either one of Claims 9 and 10, wherein in the operating position of the dispensing member (12), the first end (131) of each strip (13) is at least pressed against the inclined intermediate portion (102c), and is preferably pinched between the protruding part (121) of the dispensing member (12) and the inclined intermediate portion (102c).
